# EUROPEAN PATENT APPLICATION

(11) **EP 4 357 333 A1**
(43) Date of publication of application: **24.04.2024**
(21) Application number: 22824244.2
(22) Date of filing: 15.06.2022
(51) Int. Cl.: C07C 303/40

(54) **HYBUTIMIBE INTERMEDIATE AND PREPARATION METHOD THEREFOR**

(30) Priority: 17.06.2021 CN 202110669168
(71) Applicant: Zhejiang Hisun Pharmaceutical Co., Ltd., Taizhou, Zhejiang 318000 (CN)
(72) Inventor: LI, Xufei, Taizhou, Zhejiang 318000 (CN); WU, Jinlong, Taizhou, Zhejiang 318000 (CN); ZHANG, Yong, Taizhou, Zhejiang 318000 (CN); ZHANG, Yuncai, Taizhou, Zhejiang 318000 (CN); XU, Xiaojie, Taizhou, Zhejiang 318000 (CN); CHEN, Lei, Taizhou, Zhejiang 318000 (CN); WANG, Xiaoming, Taizhou, Zhejiang 318000 (CN); CHEN, Yanan, Taizhou, Zhejiang 318000 (CN); JIN, Meichun, Taizhou, Zhejiang 318000 (CN); ZHOU, Yaqian, Taizhou, Zhejiang 318000 (CN); ZHAN, Dongkai, Taizhou, Zhejiang 318000 (CN)
(74) Representative: Dehns
(86) International application number: PCT/CN2022/098917
(87) International publication number: WO 2022/262768

(57) **Abstract**

The present invention provides a hybutimibe intermediate and a preparation method therefor. The method comprises the steps of: reacting an easily available raw material of p-fluorobenzoyl butyrate with cheap sulfonylhydrazide to obtain sulfonylhydrazone valerate, then reacting sulfonylhydrazone valerate with carbon dioxide under an alkaline condition to obtain a compound of formula VI with a Z configuration, followed by subjecting the compound of formula VI to acylation, reduction and hydrolysis to obtain the hybutimibe intermediate of (Z)-5-(4-fluorophenyl)-6-hydroxy-hex-4-enoic acid. The preparation method involves readily available and cheap raw materials, has mild reaction conditions, simple and safe operation, a good stereoselectivity, a high yield and low costs, and is suitable for industrial mass production.

## Description

### Technical Field

The invention belongs to the field of medicinal chemistry, and specifically relates to the preparation of the key intermediate (Z)-5-(4-fluorophenyl)-6-hydroxy-hex-4-enoic acid of hybutimibe, the intermediate compound VI used for preparing the key intermediate (Z)-5-(4-fluorophenyl)-6-hydroxy-hex-4-enoic acid, and the preparation method for the intermediate formula VI compound.

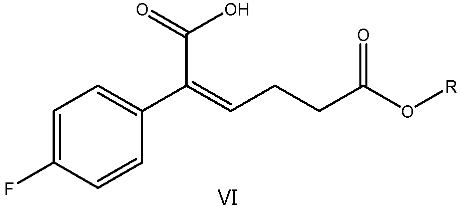

### Background

Hybutimibe (HS-25) is a cholesterol-lowering drug with the following structural formula:

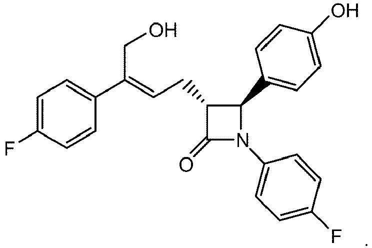

There is a trisubstituted cis-olefin structure in the structure of hybutimibe. According to organic structure theory and related experiments, the Gibbs free energy of the cis-olefin in the hybutimibe structure is higher than that of its trans isomer, that is, the trans-olefin is more stable than the desired cis-olefin. Therefore, the hybutimibe trisubstituted products obtained by conventional olefin synthesis methods, such as Wittig-Horner reaction, hydroxyl dehydration reaction, aldol condensation reaction, olefin rearrangement reaction, oxidation and other reactions are mainly trans olefins. For example, the trans-olefin/cis-olefin in the product obtained by the Wittig-Horner reaction is about 3/1, and the trans-olefin/cis-olefin in the product obtained by the aldol condensation reaction is about 9/1. Even if some synthesis methods can obtain a small amount of cis-olefins, they can easily be converted into more stable trans-olefins in the post-processing process or subsequent reaction steps. However, trans-olefins cannot be used in the commercial production of hybutimibe. Therefore, this determines that obtaining the cis-olefin structure with high selectivity is the difficulty and key point in the synthesis of hybutimibe. WO2011017907 discloses a method for preparing hybutimibe, the synthetic route of this method is too long, and it cannot stereoselectively produce cis-configured double bonds (i.e., Z-configuration, hereafter the Z-configuration will be used to represent cis-olefins), and some steps are not suitable for industrial production. WO2015188727 discloses another method for preparing hybutimibe and its key intermediate (Z)-5-(4-fluorophenyl)-6-hydroxy-hex-4-enoic acid. The structural formula of the intermediate is:

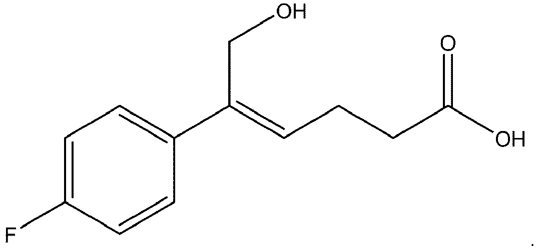

The preparation method of the key intermediate (Z)-5-(4-fluorophenyl)-6-hydroxy-hex-4-enoic acid directly affects the cost, difficulty and industrial feasibility of the entire hybutimibe production process. The preparation route of the key intermediate (Z)-5-(4-fluorophenyl)-6-hydroxy-hex-4-enoic acid of hybutimibe disclosed in WO2015188727 is: step 1: using methoxycarbonylcyclopentanone as the starting material, acetonitrile as the reaction solvent, and performing pressure catalytic oxidation with copper sulfate to obtain ring-opened ketone ester; step 2: performing an addition reaction between ring-opened ketone ester and a Grignard reagent to obtain tertiary alcohol; step 3: heating and refluxing the obtained tertiary alcohol with a dehydrating agent trifluoromethanesulfonic anhydride to obtain Z-α,β-unsaturated ester; step 4: performing a selective reduction on ester with diisobutylaluminum hydride to obtain the key intermediate (Z)-5-(4-fluorophenyl)-6-hydroxy-hex-4-enoic acid of hybutimibe. In the above preparation method disclosed in WO2015188727, the first step of pressure oxidation is a free radical reaction, which makes temperature control difficult and risky in large-scale production; the yield of the second step of Grignard addition reaction is low during scale-up production, this reaction requires a huge amount of Grignard reagents, approximately 2,000 liters of Grignard reagents are required to produce 30 kilograms of cis-olefins, and the production operation is difficult; the third and fourth steps use expensive reagents trifluoromethanesulfonic anhydride and diisobutylaluminum hydride, at the same time, the process operations are complicated and are not suitable for industrial preparation; trifluoromethanesulfonic anhydride is expensive and is a flammable and dangerous product, the post-processing of diisobutylaluminum hydride is very troublesome and is not suitable for industrial production. In short, the above existing synthesis method of the key intermediate (Z)-5-(4-fluorophenyl)-6-hydroxy-hex-4-enoic acid of hybutimibe has problems such as high risk, complicated operation, low yield, and high cost, which limits the application of this method in the large-scale production of hybutim ibe.

Therefore, it is necessary to develop a new preparation method for the key intermediate (Z)-5-(4-fluorophenyl)-6-hydroxy-hex-4-enoic acid to overcome the problems of high risk, complicated operation, low yield and high cost of the existing method.

### Summary

In order to solve the above technical problems, in the first aspect of the invention, a compound of formula VI is provided: 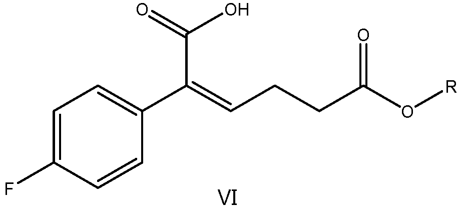 wherein, R is selected from C₁-C₇ alkyl, -CH₂CH=CH₂ or -CH₂-C₆H₅, preferably C₁-C₄ alkyl, -CH₂CH=CH₂ or -CH₂-C₆H₅, more preferably -CH₃, -CH₂CH₃, -CH₂CH₂CH₃, -CH(CH₃)₂, -C(CH₃)₃, -CH₂CH=CH₂ or -CH₂-C₆H₅.

In another aspect of the invention, a method for preparing the compound of formula VI is provided, the method comprising:
step (a): reacting a compound of formula III with a compound of formula IV to obtain a compound of formula V; 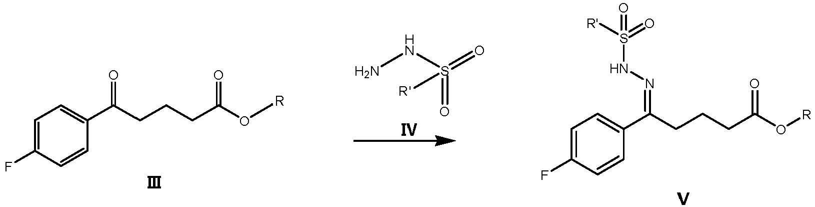
step (b): under a basic condition, heating and reacting the compound of formula V with CO₂ to obtain the compound of formula VI; 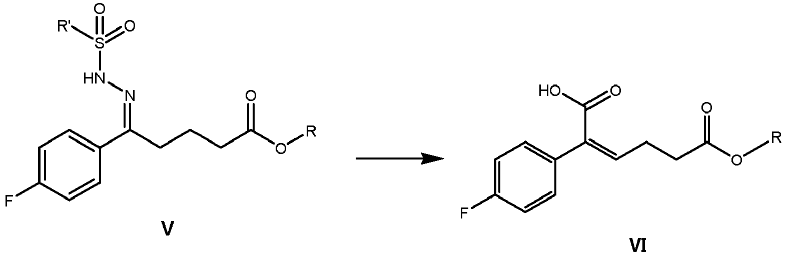
wherein, R is selected from C₁-C₇ alkyl, -CH₂CH=CH₂ or -CH₂-C₆H₅, preferably C₁-C₄ alkyl, -CH₂CH=CH₂ or -CH₂-C₆H₅, more preferably -CH₃, -CH₂CH₃, -CH₂CH₂CH₃, -CH(CH₃)₂, -C(CH₃)₃, -CH₂CH=CH₂ or -CH₂-C₆H₅; R' is alkyl, substituted or unsubstituted aryl, preferably C₁-C₃ alkyl, substituted or unsubstituted phenyl, more preferably p-tolyl or methyl, and most preferably p-tolyl.

In the above reaction steps, wherein:
a solvent used in the reaction of step (a) is selected from methanol, ethanol, n-propanol, isopropanol, allyl alcohol, benzyl alcohol or toluene, preferably methanol, ethanol, n-propanol or toluene, more preferably methanol or ethanol.

An acid used in the reaction of step (a) is an inorganic acid or an organic acid, preferably concentrated sulfuric acid, concentrated hydrochloric acid, phosphoric acid, boron trifluoride ether (BF₃·Et₂O), aluminum trioxide (Al₂O₃), trifluoroacetic acid, p-toluenesulfonic acid, methanesulfonic acid or strongly acidic cationic resin, more preferably concentrated sulfuric acid, boron trifluoride ether (BF₃·Et₂O), p-toluenesulfonic acid or strongly acidic cationic resin, more preferably concentrated sulfuric acid or p-toluenesulfonic acid.

The reaction temperature in step (a) ranges from 25°C to the reflux temperature of the solvent for the reaction, preferably the reflux temperature of the solvent for the reaction.

A solvent used in the reaction of step (b) is selected from N,N dimethylformamide (DMF), dimethyl sulfoxide (DMSO), N-methylpyrrolidone (NMP), sulfolane, dimethyl sulfone, 1,3-dimethyl-2-imidazolinone (DMI), 1,3-Dimethyl-3,4,5,6-tetrahydro-2(*1H*)-pyrimidinone (DMPU) or mixtures thereof, preferably dimethyl sulfoxide (DMSO), dimethyl sulfone, N-methylpyrrolidone (NMP), 1,3-Dimethyl-3,4,5,6-tetrahydro-2(*1H*)-pyrimidinone (DMPU) or mixtures thereof, more preferably a mixture of N-methylpyrrolidone (NMP) and dimethyl sulfone.

A base used in step (b) is an inorganic base or an organic base, the inorganic base is preferably cesium carbonate (Cs₂CO₃), potassium phosphate (K₃PO₄), potassium carbonate (K₂CO₃), sodium hydroxide, potassium hydroxide or sodium hydride (NaH), the organic base is preferably sodium methoxide, sodium ethoxide, potassium tert-butoxide, butyllithium, sodium bis(trimethylsilyl)amide, potassium bis(trimethylsilyl)amide, lithium diisopropylamide (LDA), 1,8-Diazabicyclo[5.4.0]undec-7-ene (DBU), 1,5-diazabicyclo[4.3.0]non-5-ene (DBN), triethylene diamine or 1,1,3,3-tetramethylguanidine; the base used is more preferably sodium hydride (NaH) or 1,8-Diazabicyclo[5.4.0]undec-7-ene (DBU).

The reaction temperature of the heating reaction in step (b) is 50-95°C, preferably 80-85°C.

The molar ratio of the base to the compound of formula V under the basic conditions of step (b) is 3-4.5:1, preferably 4:1; the volume of the solvent used in the reaction of step (b) is 3-6 times, preferably 3-4 times, the weight of the compound of formula V, and the unit is L/kg.

In another aspect of the present invention, a method for preparing a compound of formula VII from the compound of formula VI is provided, the method comprising: converting the compound of formula VI into an acyl halide or a mixed anhydride, and then performing a selective reduction reaction and a hydrolysis reaction to obtain a compound of formula VII: 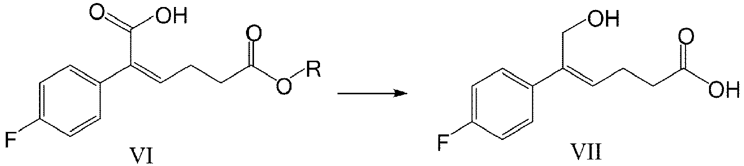 wherein, the compound of formula VI reacts with acid halide (under DMF catalysis) to form an acyl halide, or the compound of formula VI reacts with an acylating agent (in the presence of an acid-binding agent (such as triethylamine)) to form a mixed anhydride; the acid halide is preferably acid chloride, more preferably oxalyl chloride; the acylating agent is preferably chloroformate, more preferably methyl chloroformate, ethyl chloroformate, propyl chloroformate or isobutyl chloroformate;
R is selected from C₁-C₇ alkyl, -CH₂CH=CH₂ or -CH₂-C₆H₅, preferably C₁-C₄ alkyl, -CH₂CH=CH₂ or -CH₂-C₆H₅, more preferably -CH₃, -CH₂CH₃, -CH₂CH₂CH₃, -CH(CH₃)₂, -C(CH₃)₃, -CH₂CH=CH₂ or -CH₂-C₆H₅.

A solvent for the reaction between the compound of formula VI and the acid halide is selected from toluene, dichloromethane or a mixture thereof, preferably a mixture of toluene and dichloromethane; the reaction temperature of the compound of formula VI and the acid halide is 0-15°C, preferably 0-10°C; the molar ratio of the compound of formula VI to the acid halide is 1:1.1-1.3.

The reaction temperature of the compound of formula VI and the acylating agent is -20-0°C, preferably -10-0°C; the molar ratio of the compound of formula VI to the acylating agent is 1:1.1-1.3.

The reaction temperature of the reduction reaction is -60~-15°C, preferably -50~-40°C; a reducing agent for the reduction reaction is sodium borohydride or potassium borohydride, preferably sodium borohydride; the molar ratio of the reducing agent for the reduction reaction to the compound of formula VI is 1.2-1.3:1; the volume and mass ratio of methanol or ethanol for the reduction reaction to the compound of formula VI is 1.0-1.2:1 (unit: L/kg).

A base for the hydrolysis reaction is sodium hydroxide or potassium hydroxide, preferably sodium hydroxide.

In another aspect of the invention, a method for preparing a compound of formula VII from the compound of formula VI is provided, the method comprising:
step A: reacting the compound of formula VI with acid halide to form an acyl halide (i.e., a compound of formula VI-A1), or reacting the compound of formula VI with an acylating agent to form a mixed anhydride (i.e., a compound of formula VI-A2); 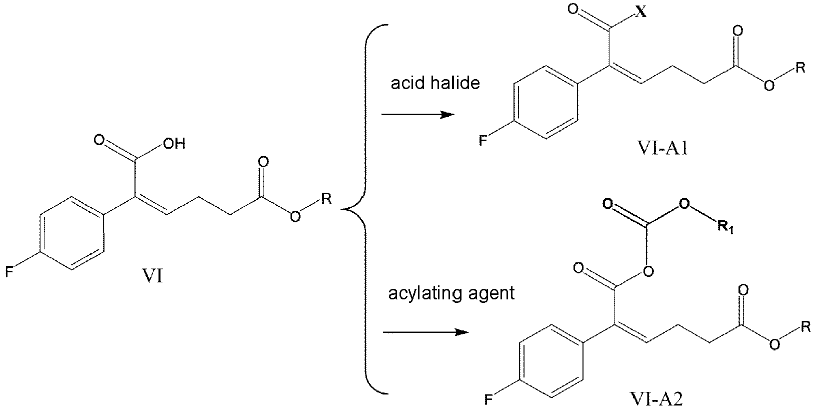
step B: performing a selective reduction reaction on the compound of formula VI-A1 or the compound of formula VI-A2 obtained in step A to obtain a compound of formula VI-B; 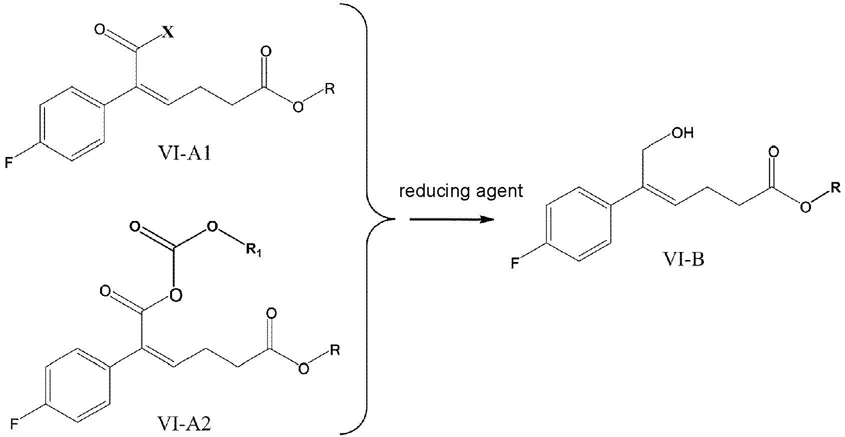
step C: performing a hydrolysis reaction on the compound of formula VI-B obtained in step B to obtain a compound of formula VII, 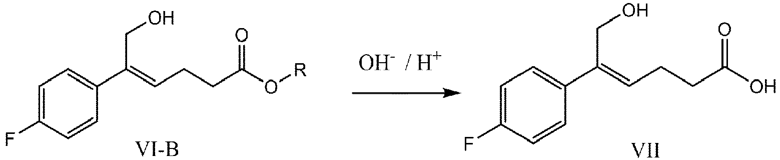
wherein, R is selected from C₁-C₇ alkyl, -CH₂CH=CH₂ or -CH₂-C₆H₅, preferably C₁-C₄ alkyl, -CH₂CH=CH₂ or -CH₂-C₆H₅, more preferably -CH₃, -CH₂CH₃, -CH₂CH₂CH₃, -CH(CH₃)₂, -C(CH₃)₃, -CH₂CH=CH₂ or -CH₂-C₆H₅; R₁ is C₁-C₄ alkyl, preferably methyl, ethyl, n-propyl or isobutyl; X is a halogen atom, preferably chloro (Cl).

A solvent for the reaction between the compound of formula VI and the acid halide in step A is selected from toluene, dichloromethane or a mixture thereof, preferably a mixture of toluene and dichloromethane; the reaction temperature of the compound of formula VI and the acid halide is 0 to 15°C, preferably 0 to 10°C; the molar ratio of the compound of formula VI to the acid halide is 1:1.1-1.3; the acid halide is acid chloride, preferably oxalyl chloride.

A solvent for the reaction between the compound of formula VI and the acylating agent in step A is selected from tetrahydrofuran, dichloromethane or a mixture thereof; the reaction temperature of the compound of formula VI and the acylating agent is -20-0°C, preferably -10-0°C; the molar ratio of the compound of formula VI to the acylating agent is 1:1.1-1.3; the acylating agent is preferably chloroformate, more preferably methyl chloroformate, ethyl chloroformate, propyl chloroformate or isobutyl chloroformate.

A solvent for the reduction reaction in step B is selected from toluene, dichloromethane or a mixture thereof; the reaction temperature of the reduction reaction is -60~-15°C, preferably -50--40°C; a reducing agent for the reduction reaction is sodium borohydride or potassium borohydride, preferably sodium borohydride; the molar ratio of the reducing agent in the reduction reaction to the compound of formula VI in step A is 1.2-1.3:1; methanol or ethanol is used to activate the reducing agent in the reduction reaction, wherein, the volume mass ratio of methanol or ethanol to the compound of formula VI in step A is 1.0-1.2:1 (unit: L/kg).

A base for the hydrolysis reaction in step C is sodium hydroxide or potassium hydroxide, preferably sodium hydroxide.

The advantages of the present invention are:
in the preparation method of the hybutimibe intermediate provided by the invention: the compound of formula V is obtained by reacting the easily available raw material p-fluorobenzoyl butyrate compound of formula III with the cheap sulfonylhydrazide compound of formula IV, the reaction conditions are mild, no dangerous reagents are used, and the product post-processing and purification methods are easy to operate; the compound of formula V reacts with CO₂ under a basic condition to obtain the compound of formula VI in the Z configuration (the ratio of the Z configuration product to the E configuration product in the obtained product is greater than 15/1), which is suitable for industrial large-scale production; in the reduction reaction, cheap and easily available sodium borohydride is used instead of expensive diisobutylaluminum hydride, and the post-processing of the sodium borohydride reaction is simple; the yield of the key intermediate (Z)-5-(4-fluorophenyl)-6-hydroxy-hex-4-enoic acid (compound of formula VII) of hybutimibe prepared by the method of the present invention is significantly higher than that of WO2015/188727 patent, and the cost is significantly reduced. In short, the preparation method provided by the present invention involves readily available and cheap raw materials and reagents, has mild reaction conditions, simple and safety operation, a good stereoselectivity, a high yield, low costs, and is suitable for industrial large-scale production.

### Detailed description

The present invention is further described below through preferred examples and specific operations, but the present invention is not limited to the scope of the examples.

Sources and specifications of raw materials and reagents:
methyl p-fluorobenzoyl butyrate (formula III-1, self-made, for the synthesis method, please refer to Applications of Disspiroketals in Synthesis, Entwistle, David Andrew, University of Cambridge, 1993), ethyl p-fluorobenzoyl butyrate (formula III-2, self-made, for the synthesis method, please refer to Applications of Disspiroketals in Synthesis, Entwistle, David Andrew, University of Cambridge, 1993), p-toluenesulfonyl hydrazide (IV-1), methanesulfonyl hydrazide (formula IV-2); DBU, NMP; solvents such as methanol, ethanol, isopropyl alcohol, sulfolane, etc.; acids such as p-toluenesulfonic acid, concentrated sulfuric acid, concentrated hydrochloric acid, etc.; bases such as cesium carbonate, potassium phosphate, sodium hydride, etc.; all are commercially available products, chemically pure or analytically pure.

The following abbreviations are used throughout the present invention:
EA: ethyl acetate
DBU: 1,8-Diazabicyclo[5.4.0]undec-7-ene
NMP: N-methylpyrrolidone
DMF: N,N dimethylformamide
DMSO: dimethyl sulfoxide
DMI: 1,3-dimethyl-2-imidazolinone
DMPU: 1,3-Dimethyl-3,4,5,6-tetrahydro-2(*1H*)-pyrimidinone LDA: lithium diisopropylamide
DBN: 1,5-diazabicyclo[4.3.0]non-5-ene
room temperature: refers to 25°C±5°C

### Example 1: Preparation of methyl 5-(4-fluorophenyl)-5-p-toluenesulfonylhydrazone-valerate (V-1)

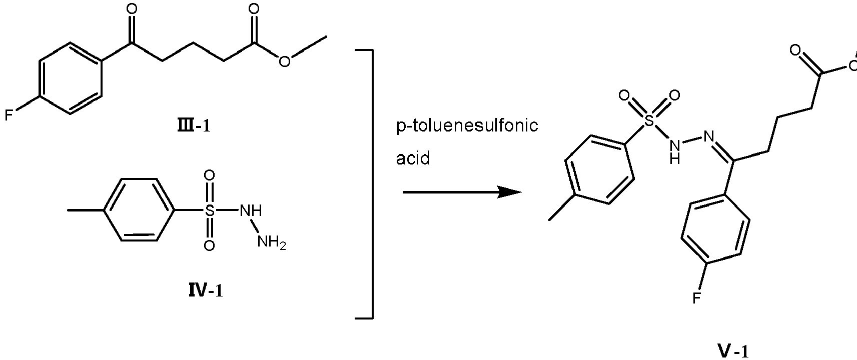

96Kg of methyl p-fluorobenzoyl butyrate (formula III-1) was added to 700L of methanol, 1Kg of p-toluenesulfonic acid and 103Kg of p-toluenesulfonyl hydrazide (IV-1) were added, and the above mixture was heated to reflux for 8h, the heating was stopped, the reaction solution was cooled to room temperature naturally, and then filtered. The filter cake was rinsed with a small amount of methanol and dried to obtain about 156Kg of methyl 5-(4-fluorophenyl)-5-p-toluenesulfonylhydrazone-valerate (V-1), with an HPLC purity of 98%, and a yield of about 93%.

¹H NMR (400 MHZ, DMSO-d6): δ 1.57 (m, 2 H), 2.37 (m, 5 H), 2.67 (m, 2 H), 3.59 (s, 3 H), 7.19-7.24 (m, 2 H), 7.42 (d, 2 H, J = 7.6 Hz), 7.67 (m, 2 H), 7.79 (d, 2 H, J = 7.8 Hz), 10.70 (s, 1 H); MS(m/z): 393 [M+H]⁺.

### Example 2: Preparation of ethyl 5-(4-fluorophenyl)-5-p-toluenesulfonylhydrazone-valerate (V-2)

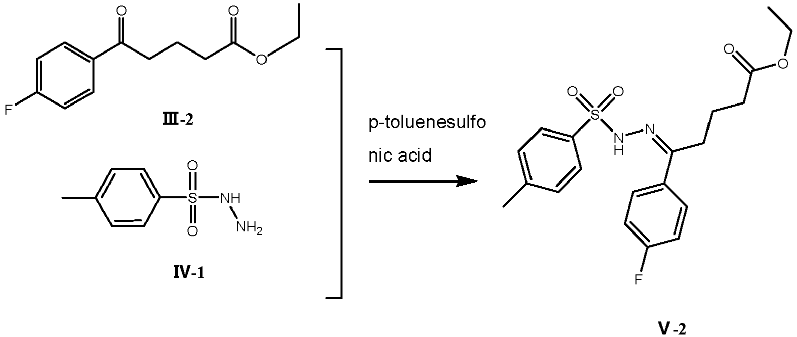

100Kg of ethyl p-fluorobenzoyl butyrate (formula III-2) was added to 700L of absolute ethanol, 1Kg of p-toluenesulfonic acid and 106Kg of p-toluenesulfonyl hydrazide (IV-1) were added, and the above mixture was heated to reflux for 8h, the heating was stopped, the reaction solution was cooled to room temperature naturally, and then filtered. The filter cake was rinsed with a small amount of ethanol and dried to obtain 146.3Kg of ethyl 5-(4-fluorophenyl)-5-p-toluenesulfonylhydrazone-valerate (V-2), with an HPLC purity of 97%, and a yield of 86%.

¹H NMR (400 MHZ, DMSO-d6): δ 1.16 (t, 3 H) 1.57 (m, 2 H), 2.37 (m, 5 H), 2.67 (m, 2 H), 4.01 (m, 2 H), 7.19-7.24 (m, 2 H), 7.42 (d, 2 H, *J* = 7.6 Hz), 7.67 (m, 2 H), 7.79 (d, 2 H, *J* = 7.8 Hz), 10.71 (s, 1 H); MS(m/z): 407 [M+H]⁺.

### Example 3: Preparation of methyl 5-(4-fluorophenyl)-5-methanesulfonylhydrazone-valerate (V-3)

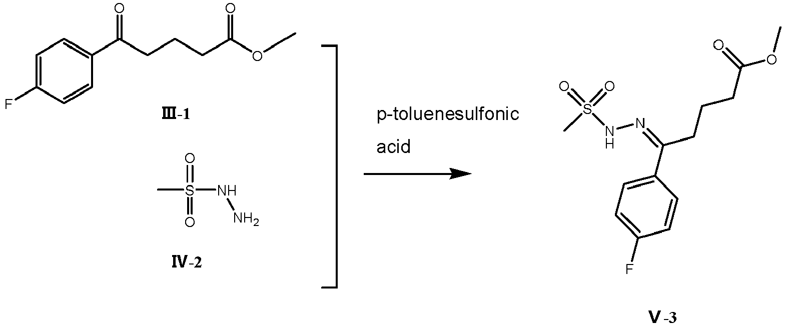

9.6g of methyl p-fluorobenzoyl butyrate (formula III-1) was added to 70ml of absolute methanol, 0.2g of p-toluenesulfonic acid and 10.3g of methanesulfonyl hydrazide (formula IV-2) were added, and the above mixture was heated to reflux for 8h, the heating was stopped, the reaction solution was cooled to room temperature naturally, and then filtered. The filter cake was rinsed with a small amount of methanol and dried to obtain 12.4g of methyl 5-(4-fluorophenyl)-5-methanesulfonylhydrazone-valerate (V-3), with an HPLC purity of 98%, and a yield of 91%.

¹H NMR (400 MHZ, DMSO-d6): δ 1.57 (m, 2 H), 2.37 (m, 2 H), 2.67 (m, 2 H), 2.75 (s, 3 H),3.58 (s, 3 H), 7.19-7.24 (m, 2 H), 7.67 (m, 2 H), 10.63 (s, 1 H); MS(m/z): 317 [M+H]⁺.

### Example 4: Preparation of Z-5-methoxycarbonyl-2-(4fluorophenyl)-2-ene-valerianic acid (VI-1)

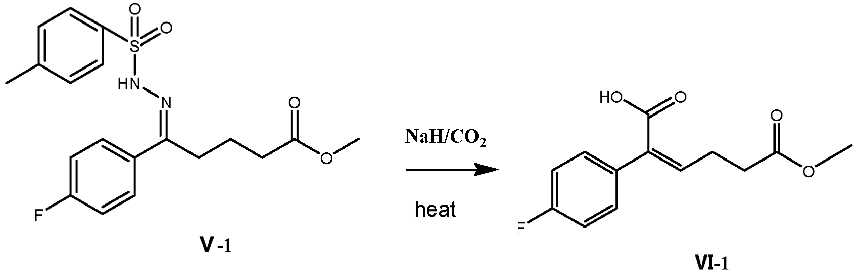

In nitrogen conditions, 180ml of DMSO and 30g of methyl 5-(4-fluorophenyl)-5-p-toluenesulfonylhydrazone-valerate (V-1) were added to the 500ml dry reaction flask, and 10 g of NaH were added in batches. After the above feedings were completed, CO₂ was introduced. The above mixture was heated to 80-85°C until the raw materials disappeared completely, then the heating was stopped and the reaction solution was cooled to room temperature. 30ml of saturated ammonium chloride aqueous solution was added to quench the reaction. The reaction solution was poured into 150ml of cold 3M dilute hydrochloric acid for acidification, extracted with ethyl acetate (20mlx3), washed with 20ml of saturated brine, and 15g/60ml of potassium carbonate aqueous solution was added to form a salt. The separated aqueous phase was acidified to pH~4 with 5M hydrochloric acid solution, extracted with 30ml of ethyl acetate, dried over anhydrous sodium sulfate, and concentrated to obtain 13g of Z-5-methoxycarbonyl-2-(4fluorophenyl)-2-ene-valerianic acid (VI-1) [HPLC purity 90%, molar yield 65%].

¹H NMR(400 MHz, DMSO-d6): δ 2.49-2.52 (m, 2 H), 2.58-2.64 (m, 2 H), 3.60 (s, 3 H), 6.15 (t, 1 H, *J* = 7.4 Hz), 7.17 (t, 2 H, *J* = 8.9 Hz), 7.32-7.37 (m, 2 H), 12.97 (br s, 1 H); MS (m/z): 252 [M+H]⁺.

### Example 5: Preparation of Z-5-methoxycarbonyl-2-(4fluorophenyl)-2-ene-valerianic acid (VI-1)

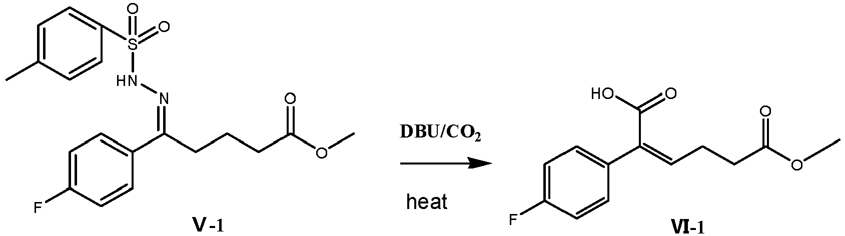

200L of N-methylpyrrolidone, 125Kg of dimethyl sulfone and 70Kg DBU were added to the 500L dry reaction tank, and then 45Kg of methyl 5-(4-fluorophenyl)-5-p-toluenesulfonylhydrazone-valerate (V-1) was added. CO₂ was continuously introduced and the mixture was heated to 80-83°C. The reaction was carried out until the disappearance of raw materials detected by HPLC, which was the end point. The above reaction solution was cooled to room temperature, and dimethyl sulfone was recovered by filtration. The filtered residue was acidified with 400L of 3M dilute hydrochloric acid, and 120L of EA was added to extract three times. The EA phase was combined, washed with 50L of saturated brine, and 60Kg/200L of potassium carbonate aqueous solution was added to form a salt. The separated aqueous phase was acidified to pH~4 with 5M hydrochloric acid, extracted with 120L of ethyl acetate, dried over anhydrous sodium sulfate, and concentrated to obtain 20.75Kg of Z-5-methoxycarbonyl-2-(4fluorophenyl)-2-ene-valerianic acid (VI-1) [HPLC purity 90%, molar yield 71%].

### Example 6: Preparation of Z-5-methoxycarbonyl-2-(4fluorophenyl)-2-ene-valerianic acid (VI-1)

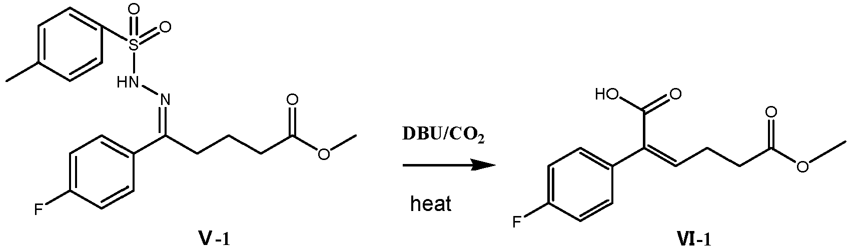

In nitrogen conditions, 150ml of N,N-dimethylformamide, 90g of dimethyl sulfone, 30g of methyl 5-(4-fluorophenyl)-5-p-toluenesulfonylhydrazone-valerate (V-1), 45g of DBU were added to the 500ml dry reaction flask. After the above feedings were completed, CO₂ was introduced. The above mixture was heated to 80-85°C until the raw materials disappeared completely, then the heating was stopped and the reaction solution was cooled to room temperature. Dimethyl sulfone was recovered by filtration. Then the filtered residue was acidified to pH~4 with cold 3M hydrochloric acid, extracted with ethyl acetate (20mlx3), washed with 20ml of saturated brine, and 15g/60ml of potassium carbonate aqueous solution was added to form a salt. The separated aqueous phase was acidified to pH~4 with 5M dilute hydrochloric acid, extracted with 30ml of ethyl acetate, dried over anhydrous sodium sulfate, and concentrated to obtain 13g of Z-5-methoxycarbonyl-2-(4fluorophenyl)-2-ene-valerianic acid (VI-1) [HPLC purity 87%, molar yield 61%].

### Example 7: Preparation of Z-5-methoxycarbonyl-2-(4fluorophenyl)-2-ene-valerianic acid (VI-1)

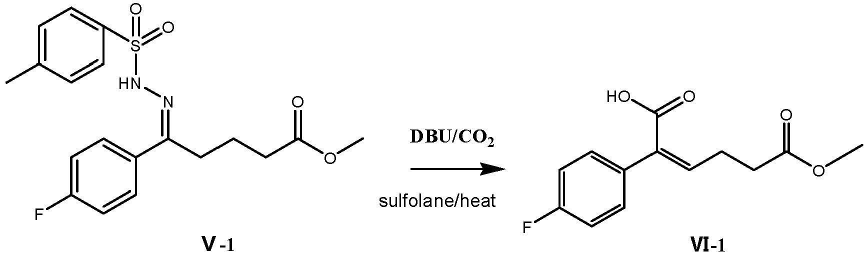

In nitrogen conditions, 150ml of sulfolane, 30g of methyl 5-(4-fluorophenyl)-5-p-toluenesulfonylhydrazone-valerate (V-1), 45g of DBU were added to the 500ml dry reaction flask. After the above feedings were completed, CO₂ was introduced. The above mixture was heated to 80-85°C until the raw materials disappeared completely, then the heating was stopped and the reaction solution was cooled to room temperature, then acidified to pH~4 with 150ml of cold 3M dilute hydrochloric acid, extracted with ethyl acetate (20mlx3), washed with 20ml of saturated brine, and 15g/60ml of potassium carbonate aqueous solution was added to form a salt. The separated aqueous phase was acidified to pH~4 with 5M hydrochloric acid, extracted with 30ml of ethyl acetate, dried over anhydrous sodium sulfate, and concentrated to obtain 30g of a sulfolane mixture of Z-5-methoxycarbonyl-2-(4fluorophenyl)-2-ene-valerianic acid (VI-1) [HPLC purity 94%].

### Example 8: Preparation of compound of formula VII

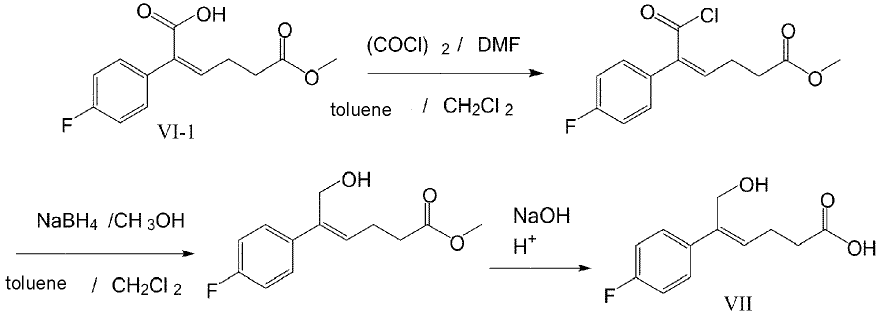

Step A: in nitrogen conditions, 100.0g(0.397mol) of Z-5-methoxycarbonyl-2-(4fluorophenyl)-2-ene-valerianic acid (compound VI-1), 600mL of toluene, 120mL of dichloromethane, 2.9g(0.0396mol) of N,N-dimethylformamide were added to the 2L reaction flask and stirred until the solution was clear. The reaction temperature was lowered and controlled at 0-10°C, and 65.5g (0.516mol) of oxalyl chloride was added dropwise. The reaction temperature was controlled at 0 to 10°C after the dropping was completed, and the reaction continued until the raw materials reacted completely. 400mL of drinking water was dropped to quench the reaction, the reaction solution was left to separate, and the aqueous phase was extracted once with 50ml of toluene. The organic phases were combined, adjusted to PH=5-6 with 1% sodium bicarbonate aqueous solution, left to separate, the organic phase was dehydrated with 10g of anhydrous magnesium sulfate and filtered to obtain an acid chloride feed liquid.

Step B: in nitrogen conditions, the acid chloride feed liquid obtained in step A was put into the 2L reaction flask, the reaction temperature was lowered to -60--50°C, and 18.0g (0.476mol) of sodium borohydride was added. The temperature was controlled at -50~-40°C, and 110mL of anhydrous methanol was added dropwise. After the dropping was completed, the temperature was controlled at -50~-40°C, and the reaction continued for 1 h. 350 mL of water was added to quench the reaction, and then the reaction solution was adjusted to pH=4-5 with dilute hydrochloric acid, and left to separate; the aqueous phase was extracted twice with dichloromethane (40ml/time), the organic phases were combined, and the volume of the reduced feed liquid was concentrated under reduced pressure to about 350-400mL; the organic phases were adjusted to pH=8-9 with 10% sodium bicarbonate aqueous solution, left to separate, and the aqueous phase was separated to obtain the reduced feed liquid (HPLC purity: 85%).

Step C: the reduced feed liquid obtained in step B was put into the 1.5L reaction flask, and 200ml of sodium hydroxide aqueous solution (preparation method: 23.8g of sodium hydroxide was dissolved in 200ml water) was added. The reaction temperature was controlled at 10-20°C, and the mixture was stirred until the raw materials reacted completely. The reaction solution was left to separate, the organic phase was extracted once with 40ml of water, the aqueous phases were combined, adjusted to pH=3-4 with dilute hydrochloric acid, and then extracted twice with ethyl acetate (150ml/time); the ethyl acetate phases were combined, washed once with 50 ml of saturated sodium chloride solution, dehydrated with 20g of anhydrous sodium sulfate, filtered, and concentrated under reduced pressure to dryness. Then a mixed solvent of toluene and ethyl acetate was added for crystallization, and the solid was filtered and dried under vacuum to obtain about 48.5g of compound VII (content: 99.50%; molar yield 54.3%).

¹H NMR (400 MHz, DMSO-d6): δ 2.37 (m, 2 H), 2.46 (m, 2 H), 4.35 (s, 2 H), 4.77 (br s, 1 H), 5.78 (t, 1 H), 7.09 (m, 2 H), 7.44 (m, 2 H), 12.16(s, 1 H); MS(m/z): 223 [M-H]⁻_{∘}

### Example 9: Preparation of compound of formula VII

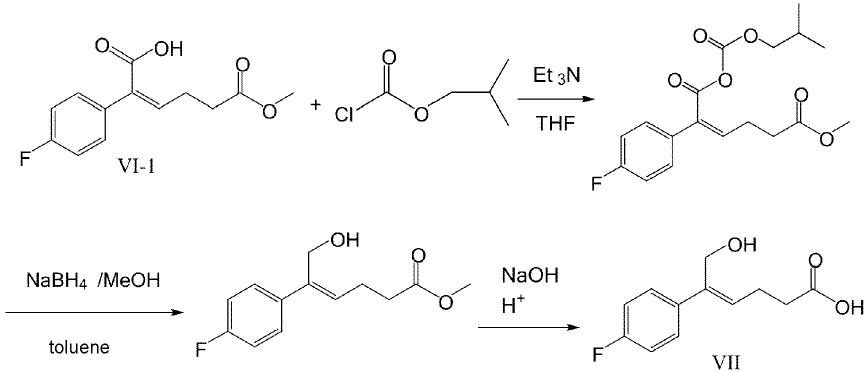

Step A: in nitrogen conditions, 10.0g(0.0397mol) of Z-5-methoxycarbonyl-2-(4fluorophenyl)-2-ene-valerianic acid (compound VI-1), 60mL of tetrahydrofuran, 5.2g(0.0515mol) of triethylamine were added to the 200ml reaction flask and stirred until the solution was clear. The reaction temperature was lowered and controlled at -10~0°C, and 6.0g(0.0439mol) of isobutyl chloroformate was added dropwise. After the dropping was completed, the mixture was stirred until the raw materials reacted completely. The reaction solution was filtered, and the resulting mother liquor was concentrated to dryness under reduced pressure to obtain a mixed anhydride.

Step B: in nitrogen conditions, the mixed anhydride obtained in step A was put into the 200ml reaction flask, 60ml of toluene was added and the mixture was stirred until the solution was clear. The reaction temperature was lowered to -50~-40°C, and 1.8g (0.0476mol) of sodium borohydride was added. The temperature was controlled at -50~-40°C, and 12mL of methanol was added dropwise. After the dropping was completed, the temperature was controlled at -50~-40°C, and the reaction continued for 0.5 h. 35 mL of water was added to quench the reaction, and then the reaction solution was adjusted to pH=4-5 with dilute hydrochloric acid, and left to separate, the aqueous phase was extracted twice with toluene (5ml/time), the organic phases were combined, and the volume of the reduced feed liquid was concentrated under reduced pressure to about 50mL; the organic phases were adjusted to pH=8-9 with 10% sodium bicarbonate aqueous solution, left to separate, and the aqueous phase was separated to obtain the reduced feed liquid (HPLC purity: 70%).

Step C: the reduced feed liquid obtained in step B was put into the 200ml reaction flask, and 20ml of sodium hydroxide aqueous solution (preparation method: 2.4g of sodium hydroxide was dissolved in 20ml water) was added. The reaction temperature was controlled at 10-20°C, and the mixture was stirred until the raw materials reacted completely. The reaction solution was left to separate, the organic phase was extracted once with 10ml of water, the aqueous phases were combined, adjusted to pH=3-4 with dilute hydrochloric acid, and then extracted twice with ethyl acetate (20ml/time); the ethyl acetate phases were combined, washed once with 10 ml of saturated sodium chloride solution, dehydrated with 3.5g of anhydrous sodium sulfate, filtered, and concentrated under reduced pressure to dryness. Then a mixed solvent of toluene and ethyl acetate was added for crystallization, and the solid was filtered and dried under vacuum to obtain about 3.7g of compound VII (content: 98.2%; molar yield 40.9%).

### Example 10: Preparation of compound of formula VII

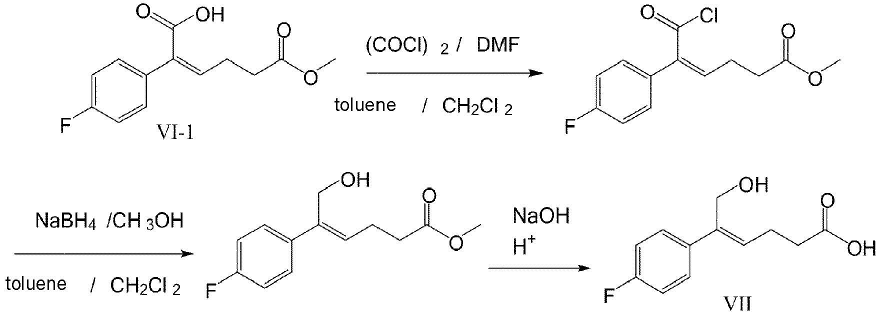

Step A: in nitrogen conditions, 50.0kg (HPLC purity 90%, 178.6mol) of Z-5-methoxycarbonyl-2-(4fluorophenyl)-2-ene-valerianic acid(compound VI-1), 300L of toluene, 60L of dichloromethane, 1.4kg of N,N-dimethylformamide were added to the 1000L reaction kettle and stirred until the solution was clear. The reaction temperature was lowered and controlled at 0-10°C, and 24.9kg(196.2mol) of oxalyl chloride was added dropwise. The reaction temperature was controlled at 0 to 10°C after the dropping was completed, and the reaction continued until the raw materials reacted completely. 200L of drinking water was dropped to quench the reaction, the reaction solution was left to separate, and the aqueous phase was extracted once with 20L of toluene. The organic phases were combined, adjusted to pH=5-6 with 1% sodium bicarbonate aqueous solution, left to separate, the organic phase was dehydrated with 5kg of anhydrous magnesium sulfate and filtered to obtain an acid chloride feed liquid.

Step B: in nitrogen conditions, the acid chloride feed liquid obtained in step A was put into the 1000L stainless steel reaction kettle, the reaction temperature was lowered to -60--50°C, and 8.1kg(214.1mol) of sodium borohydride was added. The temperature was controlled at -50--40°C, and 55L of anhydrous methanol was added dropwise. After the dropping was completed, the temperature was controlled at -50~-40°C, and the reaction continued for 1 h. 200L of water was added to quench the reaction, and then the reaction solution was adjusted to pH=4-5 with dilute hydrochloric acid, and left to separate; the aqueous phase was extracted twice with dichloromethane (20L/time), the organic phases were combined, and the volume of the reduced feed liquid was concentrated under reduced pressure to about 170-200L; the organic phases were adjusted to pH=8-9 with 10% sodium bicarbonate aqueous solution, left to separate, and the aqueous phase was separated to obtain the reduced feed liquid (HPLC purity: 83.5%).

Step C: the reduced feed liquid obtained in step B was put into the 500L reaction kettle, and 100L of sodium hydroxide aqueous solution (preparation method: 10.5kg of sodium hydroxide was dissolved in 130L water) was added. The reaction temperature was controlled at 10-20°C, and the mixture is stirred until the raw materials reacted completely. The reaction solution was left to separate, the organic phase was extracted once with 30L of water, the aqueous phases were combined, adjusted to pH=3-4 with dilute hydrochloric acid, and then extracted twice with ethyl acetate (70L/time); the ethyl acetate phases were combined, washed once with 50L of saturated sodium chloride solution, dehydrated with 8kg of anhydrous sodium sulfate, filtered, and concentrated under reduced pressure to dryness. Then a mixed solvent of toluene and ethyl acetate was added for crystallization, and the solid was filtered and dried under vacuum to obtain about 19.5kg of compound VII (content: 99.40%; molar yield 54.8%).

## Claims

1. A compound of formula VI: 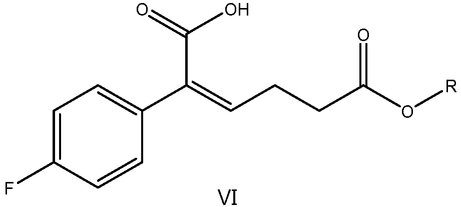 wherein, R is selected from C₁-C₇ alkyl, -CH₂CH=CH₂ or -CH₂-C₆H₅, preferably C₁-C₄ alkyl, -CH₂CH=CH₂ or -CH₂-C₆H₅, more preferably -CH₃, -CH₂CH₃, -CH₂CH₂CH₃, -CH(CH₃)₂, -C(CH₃)₃, -CH₂CH=CH₂ or -CH₂-C₆H₅.

2. A method for preparing the compound of formula VI according to claim 1, the method comprising:
step (a): reacting a compound of formula III with a compound of formula IV to obtain a compound of formula V; 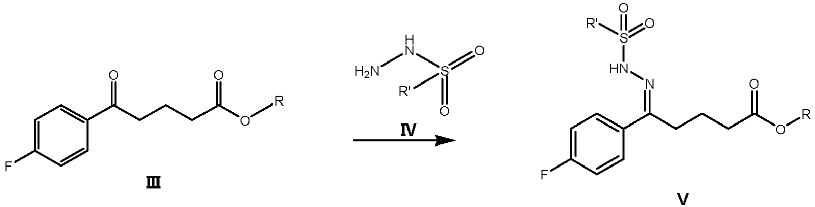
step (b): under a basic condition, heating and reacting the compound of formula V with CO₂ to obtain the compound of formula VI; 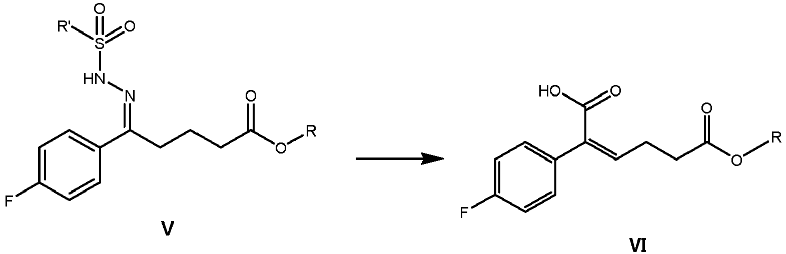
wherein, R is as defined in claim 1; R' is alkyl, substituted or unsubstituted aryl, preferably C₁-C₃ alkyl, substituted or unsubstituted phenyl, more preferably p-tolyl or methyl, and most preferably p-tolyl.

3. The method according to claim 2, wherein, a solvent used in the reaction of step (a) is selected from methanol, ethanol, n-propanol, isopropanol, allyl alcohol, benzyl alcohol or toluene, preferably methanol, ethanol, n-propanol or toluene, more preferably methanol or ethanol.

4. The method according to claim 2, wherein, an acid used in the reaction of step (a) is an inorganic acid or an organic acid, preferably concentrated sulfuric acid, concentrated hydrochloric acid, phosphoric acid, boron trifluoride ether (BFs • Et₂O), aluminum trioxide (Al₂O₃), trifluoroacetic acid, p-toluenesulfonic acid, methanesulfonic acid or strongly acidic cationic resin, more preferably concentrated sulfuric acid, boron trifluoride ether (BFs • Et₂O), p-toluenesulfonic acid or strongly acidic cationic resin, more preferably concentrated sulfuric acid or p-toluenesulfonic acid.

5. The method according to claim 2, wherein, the reaction temperature in step (a) ranges from 25°C to the reflux temperature of the solvent for the reaction, preferably the reflux temperature of the solvent for the reaction.

6. The method according to any one of claims 2-5, wherein, a solvent used in the reaction of step (b) is selected from N,N dimethylformamide (DMF), dimethyl sulfoxide (DMSO), N-methylpyrrolidone (NMP), sulfolane, dimethyl sulfone, 1,3-dimethyl-2-imidazolinone (DMI), 1,3-Dimethyl-3,4,5,6-tetrahydro-2(*1H*)-pyrimidinone (DMPU) or mixtures thereof, preferably dimethyl sulfoxide (DMSO), dimethyl sulfone, N-methylpyrrolidone (NMP), 1,3-Dimethyl-3,4,5,6-tetrahydro-2(*1H*)-pyrimidinone (DMPU) or mixtures thereof, more preferably a mixture of N-methylpyrrolidone (NMP) and dimethyl sulfone.

7. The method according to any one of claims 2-5, wherein, a base used in step (b) is an inorganic base or an organic base, the inorganic base is selected from cesium carbonate (Cs₂CO₃), potassium phosphate (K₃PO₄), potassium carbonate (K₂CO₃), sodium hydroxide, potassium hydroxide or sodium hydride (NaH), the organic base is selected from sodium methoxide, sodium ethoxide, potassium tert-butoxide, butyllithium, sodium bis(trimethylsilyl)amide, potassium bis(trimethylsilyl)amide, lithium diisopropylamide (LDA), 1,8-Diazabicyclo[5.4.0]undec-7-ene (DBU), 1,5-diazabicyclo[4.3.0]non-5-ene (DBN), triethylenediamine or 1,1,3,3-tetramethylguanidine; the base used is preferably sodium hydride (NaH) or 1,8-Diazabicyclo[5.4.0]undec-7-ene (DBU).

8. The method according to any one of claims 2-5, wherein, the reaction temperature of the heating reaction in step (b) is 50-95°C, preferably 80-85°C.

9. The method according to any one of claims 2-5, wherein, the molar ratio of the base to the compound of formula V under the basic conditions of step (b) is 3-4.5:1, preferably 4:1; the volume of the solvent used in the reaction of step (b) is 3-6 times, preferably 3-4 times, the weight of the compound of formula V, and the unit is L/kg.

10. A method for preparing a compound of formula VII from the compound of formula VI according to claim 1, the method comprising: converting the compound of formula VI into an acyl halide or a mixed anhydride, and then performing a selective reduction reaction and a hydrolysis reaction to obtain a compound of formula VII: 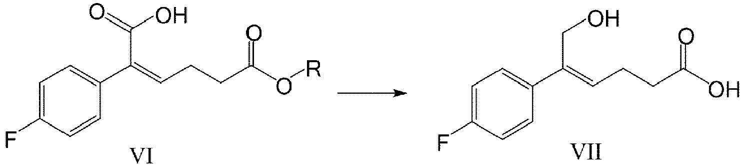 wherein, the compound of formula VI reacts with acid halide to form an acyl halide, or the compound of formula VI reacts with an acylating agent to form a mixed anhydride; the acid halide is preferably acid chloride, more preferably oxalyl chloride; the acylating agent is preferably chloroformate, more preferably methyl chloroformate, ethyl chloroformate, propyl chloroformate or isobutyl chloroformate;
R is as defined in claim 1.

11. The method according to claim 10, wherein a solvent for the reaction between the compound of formula VI and the acid halide is selected from toluene, dichloromethane or a mixture thereof, preferably a mixture of toluene and dichloromethane; the reaction temperature of the compound of formula VI and the acid halide is 0-15°C, preferably 0-10°C; the molar ratio of the compound of formula VI to the acid halide is 1:1.1 -1.3.

12. The method according to claim 10, wherein the reaction temperature of the compound of formula VI and the acylating agent is -20-0°C, preferably -10-0°C; the molar ratio of the compound of formula VI to the acylating agent is 1:1.1-1.3.

13. The method according to any one of claims 10-12, wherein the reaction temperature of the reduction reaction is -60~-15°C, preferably -50~-40°C; a reducing agent for the reduction reaction is sodium borohydride or potassium borohydride, preferably sodium borohydride; the molar ratio of the reducing agent for the reduction reaction to the compound of formula VI is 1.2-1.3:1; the volume and mass ratio of methanol or ethanol for the reduction reaction to the compound of formula VI is 1.0-1.2:1 (unit: L/kg).

14. The method according to any one of claims 10-13, wherein a base for the hydrolysis reaction is sodium hydroxide or potassium hydroxide, preferably sodium hydroxide.

15. A method for preparing a compound of formula VII from the compound of formula VI according to claim 1, the method comprising:
step A: reacting the compound of formula VI with acid halide to form an acyl halide (i.e., a compound of formula VI-A1), or reacting the compound of formula VI with an acylating agent to form a mixed anhydride (i.e., a compound of formula VI-A2); 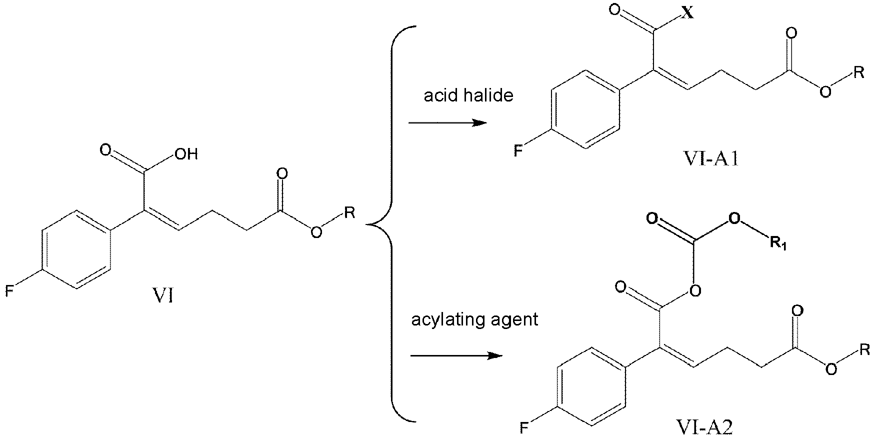
step B: performing a selective reduction reaction on the compound of formula VI-A1 or the compound of formula VI-A2 obtained in step A to obtain a compound of formula VI-B; 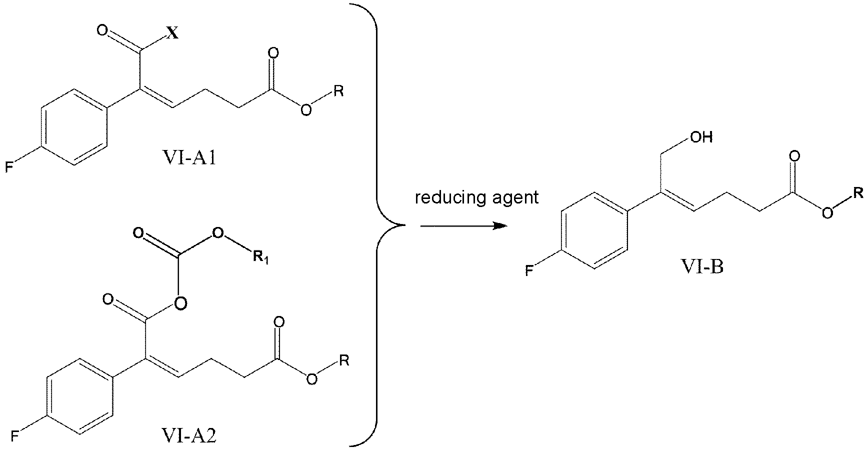
step C: performing a hydrolysis reaction on the compound of formula VI-B obtained in step B to obtain a compound of formula VII, 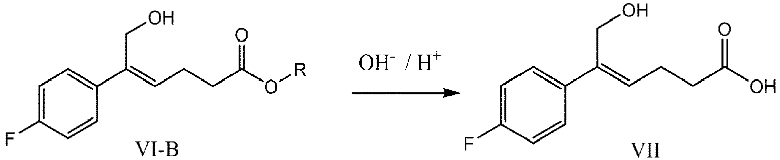
wherein, R is as defined in claim 1; R1 is C₁-C₄ alkyl, preferably methyl, ethyl, n-propyl or isobutyl; X is a halogen atom, preferably chloro (Cl).

16. The method according to claim 15, wherein, a solvent for the reaction between the compound of formula VI and the acid halide in step A is selected from toluene, dichloromethane or a mixture thereof, preferably a mixture of toluene and dichloromethane; the reaction temperature of the compound of formula VI and the acid halide is 0 to 15°C, preferably 0 to 10°C; the molar ratio of the compound of formula VI to the acid halide is 1:1.1-1.3; the acid halide is acid chloride, preferably oxalyl chloride.

17. The method according to claim 15, wherein, a solvent for the reaction between the compound of formula VI and the acylating agent in step A is selected from tetrahydrofuran, dichloromethane or a mixture thereof; the reaction temperature of the compound of formula VI and the acylating agent is -20-0°C, preferably -10-0°C; the molar ratio of the compound of formula VI to the acylating agent is 1:1.1-1.3; the acylating agent is preferably chloroformate, more preferably methyl chloroformate, ethyl chloroformate, propyl chloroformate or isobutyl chloroformate.

18. The method according to any one of claims 15-17, wherein, a solvent for the reduction reaction in step B is selected from toluene, dichloromethane or a mixture thereof; the reaction temperature of the reduction reaction is -60~-15°C, preferably -50~-40°C; a reducing agent for the reduction reaction is sodium borohydride or potassium borohydride, preferably sodium borohydride; the molar ratio of the reducing agent in the reduction reaction to the compound of formula VI in step A is 1.2-1.3:1; methanol or ethanol is used to activate the reducing agent in the reduction reaction, wherein, the volume mass ratio of methanol or ethanol to the compound of formula VI in step A is 1.0-1.2:1 (unit: L/kg).

19. The method according to any one of claims 15-18, wherein a base for the hydrolysis reaction in step C is sodium hydroxide or potassium hydroxide, preferably sodium hydroxide.
